# EUROPÄISCHE PATENTANMELDUNG

(11) **EP 1 106 597 A1**
(43) Veröffentlichungstag der Anmeldung: **13.06.2001**
(21) Anmeldenummer: 00125625.4
(22) Anmeldetag: 23.11.2000
(51) Int. Cl.: C07C 45/74, C07C 45/62, C07C 45/73, C07C 49/203, C07C 49/04

(54) **Verfahren zur Herstellung von Ketonen, insbesondere von 6-Methylheptan-2-On**

(30) Priorität: 07.12.1999 DE 19959053
(71) Anmelder: BASF AKTIENGESELLSCHAFT, 67056 Ludwigshafen (DE)
(72) Erfinder: Kramer, Andreas, Dr., 67098 Bad Dürkheim (DE); Knoll, Christian, Dr., 67141 Neuhofen (DE); Melder, Johann-Peter, Dr., 67459 Böhl-Iggelheim (DE); Siegel, Wolfgang, Dr., 67117 Limburgerhof (DE); Kaibel, Gerd, Dr., 68623 Lampertheim (DE)

(57) **Zusammenfassung**

Verfahren zur Herstellung von Ketonen durch eine sogenannte "gekreuzte Aldolkondensation" von einem Keton mit einem Aldehyd in Gegenwart eines Katalysatorsystems bestehend aus etwa äquimolaren Mengen an einem sekundären Amin und einer Carbonsäure enthaltend mindestens 2 C-Atome, unter Bildung eines α,β-ungesättigten Ketons und ggf. anschließende katalytische Hydrierung.

Das Verfahren dient insbesondere zur Herstellung von 6-Methyl-3-hepten-2-on durch Umsetzen von Aceton mit Isovaleraldehyd, bzw. zur Herstellung von dessen Hydrierungsprodukt, dem 6-Methylheptan-2-on, welches als Vorprodukt für zahlreiche Wirkstoffe, insbesondere für die Herstellung fettlöslicher Vitamine, wie Vitamin E, Bedeutung hat.

## Beschreibung

Die Erfindung betrifft ein Verfahren zur Herstellung von Ketonen durch eine sogenannte "gekreuzte Aldolkondensation" von einem Keton mit einem Aldehyd in Gegenwart eines Katalysatorsystems bestehend aus etwa äquimolaren Mengen an einem sekundären Amin und einer Carbonsäure, unter Bildung eines α,β-ungesättigten Ketons und ggf. anschließende katalytische Hydrierung.

Das Verfahren dient insbesondere zur Herstellung von 6-Methyl-3-hepten-2-on, durch Umsetzen von Aceton mit Isovaleraldehyd, bzw. zur Herstellung des Hydrierungsproduktes von 6-Methyl-3-hepten-2-on, dem 6-Methyl-heptan-2-on (MHA), welches als Vorprodukt für zahlreiche Wirkstoffe, insbesondere für die Herstellung fettlöslicher Vitamine, wie Vitamin E, Bedeutung hat.

Die Verwendung der Aldol-Reaktion zum Aufbau höherer Ketone oder Aldehyde ist in der organischen Synthese weit verbreitet und wird in der wissenschaftlichen Literatur umfangreich dokumentiert. Einen Überblick hierzu erhält man u.a. durch das Werk Houben-Weyl, *Methoden der organischen Chemie,* Band 7/1, 4. Auflage, 1979, Seite 77 f. und Band 7/2b Seite 1449 f.

Neben Säure-katalysierten Varianten sind Methoden zur Aldolkondensation unter Basenkatalyse bekannt, wobei vielfach die Hydroxide der Alkali- und Erdalkalimetalle oder auch basische Ionentauscher eingesetzt werden (vgl. oben zitierte Literatur).

Auch die Verwendung von Aminen als Katalysator für die Aldolkondensation ist in der Literatur schon beschrieben worden (vgl. Houben-Weyl, *Methoden der organischen Chemie,* Band 7/1, 4. Auflage, 1979, Seiten 87 f. und Band 7/2b Seiten 1452 f.).

Nachteilig bei der Aldolkondensation ist, insbesondere dann, wenn die Aldolkondensation beispielsweise zwischen einem Keton und einem Aldehyd (also als eine sogenannte "gekreuzte Aldolkondensation") ausgeführt wird, daß die Selektivität zu wünschen übrig läßt; da hierbei die Reaktion der Reaktanten mit sich selbst häufig in größerem Umfang erfolgt. Dieses Problem kann in vielen Fällen durch die gezielte Herstellung eines Enamins eines der Reaktionskomponenten und dessen weitere Umsetzung gelöst werden, wie beispielsweise von H. D. Engels et al. in Chem. Ber. 95 (1962), Seiten 1495-1504, oder in dem Referat von Dokl. Akad.

Nauk SSSR, 149 (1963), Seite 94, beschrieben worden ist. Allerdings wird auf diese Weise die höhere Selektivität durch die Notwendigkeit einer weiteren Synthesestufe inklusive der entsprechenden Aufarbeitungsoperationen erkauft.

Die Durchführung dieser Verfahrensvariante ohne separate Isolierung des Enamins gelingt zwar in Gegenwart einer Säure, dies allerdings nach Ergebnissen von K. Eiter in Ann. 658 (1962), Seiten 91-99, nur in schlechten Ausbeuten, wenn beide Reaktionspartner zur Bildung eines Enamins befähigt sind.

Dementsprechend wurde in den Patenten US 5,214,151 und EP 0 771 780 auch nur die Aldolkondensation von Aceton mit aromatischen Aldehyden, also Aldehyden, die nicht zur Bildung von Enaminen fähig sind, in Gegenwart eines sekundären Amins beschrieben und beansprucht.

Ein komplexes Katalysatorsystem, bestehend aus einem sekundären Amin, einer Halogensäure und einer Carbonsäure wird in dem Verfahren gemäß EP 0 429 603 B1 zur Addition von Formaldehyd, insbesondere von Paraformaldehyd, an Ketone beansprucht.

Auch die Herstellung von 6-Methyl-3-hepten-2-on durch eine Aldolkondensation von Aceton mit Isovaleraldehyd in Gegenwart von wässriger Natronlauge in relativ guter Ausbeute ist schon lange bekannt (vgl. Berichte 33 (1900), Seiten 559-566, insbesondere Seite 561).

Eine Möglichkeit zur direkten technischen Herstellung von MHA und ähnlichen Ketonen bot sich beispielsweise durch das Verfahren gemäß DE 26 15 308, bei dem man ein aliphatisches Keton, vorzugsweise Aceton, mit einem aliphatischen Aldehyd, beispielsweise Isovaleraldehyd, in Gegenwart von Wasserstoff und einem Katalysatorsystem, welches sowohl die Kondensation von Keton und Aldehyd als auch die anschließende Hydrierung katalysiert, bei Temperaturen von 80-280°C umsetzen kann. Das Verfahren wird mit Vorteil kontinuierlich an einem Festbettkatalysator durchgeführt. Bei der technischen Durchführung sind isolierbare Ausbeuten von mehr als 80 % der Theorie erreichbar. Nachteilig an diesem Verfahren ist allenfalls, daß man relativ hohe Temperaturen anwenden muß, wodurch die Gefahr besteht, durch Überhydrierung unerwünschte Nebenprodukte zu erhalten.

In EP 765 853 A1 wird ein Verfahren zur Herstellung von MHA durch Aldolkondensation beschrieben, gemäß dem Aceton und Isovaleraldehyd in Gegenwart einer basischen Verbindung zu 4-Hydroxy-6-methyl-heptan-2-on kondensiert und das erhaltene Kondensationsprodukt unter dehydratisierenden Bedingungen hydriert wird. Nachteilig an dem Verfahren ist insbesondere, daß die Kondensation im ersten Verfahrensschritt nur mit unzureichenden Ausbeuten verläuft. So werden ausweislich der Beispiele nach der Kondensation gaschromatographisch nur Ausbeuten an 4-Hydroxy-6-methyl-heptan-2-on und dem direkt gebildeten 6-Methyl-3-hepten-6-on von zusammen 76,1% in Beispiel 1 bzw. 80,6 % in Beispiel 2 bestimmt. Da hiervon durch aufwendige Aufarbeitungsschritte noch Wertprodukt verloren geht, und bei der anschließenden Hydrierung unter dehydratisierenden Bedingungen nachweislich der Beispiele nur Ausbeuten von maximal 92 % erzielt werden, können mit diesem Verfahren trotz aufwendiger Verfahrensführung nur Ausbeuten von maximal 74 % erreicht werden, was für ein großtechnisches Verfahren unzureichend ist.

Aus EP 816 321 A1 ist ein Verfahren zur Herstellung zur Herstellung von 6-Methyl-3-hepten-2-on durch gekreuzte Aldolkondensation bekannt, bei dem man Isovaleraldehyd und wäßriges Alkali, enthaltend eine basische Substanz, bei erhöhter Temperatur kontinuierlich in überschüssiges Aceton einträgt. Nachteilig an diesem Verfahren ist, daß die durch gaschromatographische Analyse bestimmte Ausbeute an 6-Methyl-3-hepten-2-on mit 66% der Theorie absolut unbefriedigend ist.

Weiterhin ist aus Anspruch 11 von EP 816 321 A1 ein Verfahren zur Herstellung von 6-Methyl-heptan-2-on oder dessen Homologen bekannt, bei dem Wasserstoff, Aceton und ein Aldehyd in Gegenwart von wässrigem Alkali, enthaltend eine basische Substanz, und einem üblichen Hydrierkatalysator umgesetzt werden sollen. Nachteilig an diesem Verfahren ist, daß in der Mehrzahl der Beispiele für dies Verfahren nur gaschromatographisch bestimmte Selektivitäten an 6-Methyl-heptan-2-on von weniger als 70 % der Theorie erzielt werden. Bei dem einzigen vorteilhafteren Beispiel beträgt die Selektivität nach der Aufarbeitung nur etwa 82 %.

Es war daher die Aufgabe der Erfindung, ein Verfahren zur großtechnischen Herstellung von Ketonen, insbesondere von 6-Methyl-3-hepten-2-on, durch gekreuzte Aldolkondensation von Aceton, mit einem aliphatischen Aldehyd, insbesondere mit Isovaleraldehyd, und gegebenenfalls anschließende Hydrierung zu 6-Methyl-heptan-2-on zu entwickeln, das es erlaubt das Keton großtechnisch auch ohne Anwendung sehr hoher Temperaturen und ohne aufwendige Aufarbeitungsschritte während der Ketonsynthese und mit sehr guten Selektivitäten herzustellen.

Es wurde nun überraschenderweise gefunden, daß man auch bei Ketonen und Aldehyden, die unter den Bedingungen einer Aldolkondensation mit sich selbst kondensieren können und die beide zur Bildung eines Enamins befähigt sind, wie Aceton und Isovaleraldehyd, in guten Ausbeuten und mit sehr guten Selektivitäten eine gekreuzte Aldolkondensation zwischen dem Aldehyd und dem Keton durchführen kann, wenn man die Umsetzung in Gegenwart eines Katalysatorsystems durchführt, das aus einem speziellen sekundären Amin, wie Dimethylamin oder Pyrrolidin, und einer Carbonsäure enthaltend mindestens 2 C-Atome, insbesondere Essigsäure, Adipinsäure oder Phthalsäure, besteht. Das zunächst bei Aldolkondensationen entstehende β-Hydroxy-keton wird dabei im Reaktionsaustrag nur noch in sehr geringen Mengen gefunden.

Das neue Verfahren wird mit Vorteil so durchgeführt, daß man zunächst überschüssiges Keton, mit dem sekundären Amin und der Carbonsäure in Gegenwart von Wasser vorlegt und erhitzt und dann den Aldehyd, beispielsweise Isovalerianaldehyd, langsam zufügt. Durch diese Reaktionsführung können α,β-ungesättigte Ketone mit Selektivitäten von mehr als 92 % hergestellt werden. Der Umsatz bezogen auf den im Unterschuß eingesetzten Aldehyd ist dabei quantitativ.

Gegenstand der Erfindung ist dementsprechend ein Verfahren zur Herstellung von α,β-ungesättigten Ketonen der allgemeinen Formel I 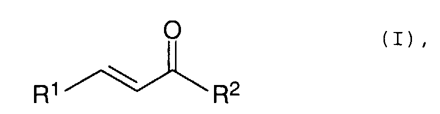 in der R¹ für einen unverzweigten oder verzweigten cyclischen oder acyclischen gesättigten oder ungesättigten aliphatischen Rest mit 4 bis 20 C-Atomen steht,
und R² für einen verzweigten oder unverzweigten cyclischen oder acyclischen gesättigten oder ungesättigten aliphatischen Rest mit 1 bis 10 C-Atomen steht,
durch Umsetzen von einem Keton der allgemeinen Formel II 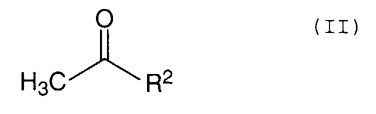 mit einem Aldehyd der allgemeinen Formel III 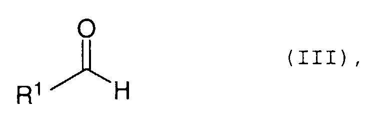 in der R¹ die oben angegebene Bedeutung hat, das dadurch gekennzeichnet ist, daß man das Keton in Gegenwart von einem Katalysatorsystem bestehend aus Dimethylamin oder Pyrrolidin und einer Carbonsäure enthaltend mindestens 2 C-Atome, vorzugsweise einer Carbonsäure enthaltend 2 bis 10 C-Atome, insbesondere Essigsäure, Adipinsäure oder Phthalsäure unter Rückfluß zum Sieden erhitzt und zu dieser Mischung langsam den Aldehyd der allgemeinen Formel III zufügt.

Es waren zwar aus EP 0 058 927 B1 und EP 0 092 097 B1 schon Verfahren zur Herstellung von α-Alkyl-acroleinen durch Umsetzen von Alkanalen mit Formaldehyd und sekundären Aminen in Gegenwart von Carbonsäuren oder Dicarbonsäuren bekannt, jedoch erfolgt bei diesen auf der Mannich-Reaktion basierenden Verfahren die Aktivierung des Formaldehyds als Akzeptorkomponente mit Hilfe des Amins gemäß dem nachfolgenden Schema 1 (vgl. Organikum, 16. Auflage (1986), Seiten 466f.).

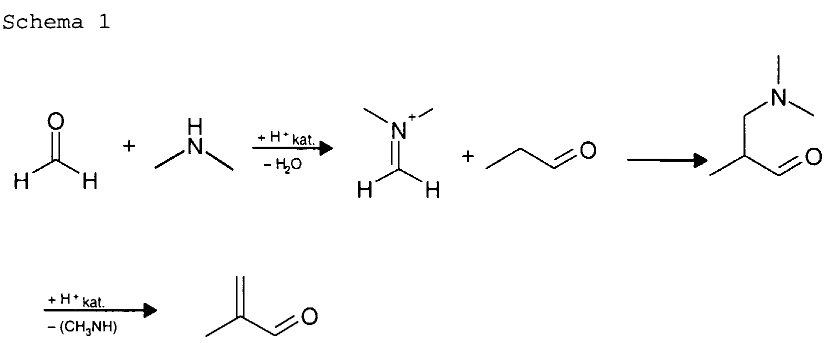

Dagegen zeichnet sich das hier beschriebene Verfahren dadurch aus, daß gemäß Schema 2 die Donorkomponente durch die Bildung eines intermediären Enamins aktiviert wird.

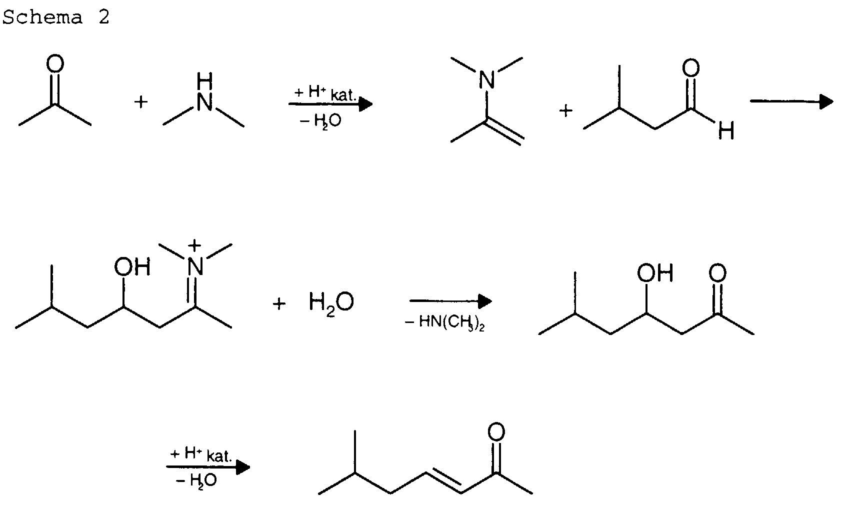

Zur Erzielung guter Umsätze und guter Selektivitäten ist es wichtig, daß man das Keton, insbesondere das Aceton, in einem molaren Überschuß gegenüber dem Aldehyd der Formel III verwendet. Als vorteilhaft haben sich Mengen an Keton von mindestens 2 Mol pro Mol des Aldehyds der Formel III, vorzugsweise etwa 4 bis 8 Mol, insbesondere etwa 5 Mol pro Mol des Aldehyds der Formel III erwiesen.

Das erfindungsgemäße Verfahren gelingt besonders vorteilhaft, wenn man in dem Katalysatorsystem das Dimethylamin oder das Pyrrolidin und die Carbonsäure in etwa äquimolaren Mengen einsetzt und dieses Katalysatorsystem in Mengen von etwa 0,5 bis 10 Mol-%, vorzugsweise 7 bis 8 Mol-%, insbesondere etwa 7,5 Mol-%, bezogen auf den im allgemeinen im molaren Unterschuß eingesetzten Aldehyd der allgemeinen Formel III verwendet.

Besonders gute Selektivitäten an dem ungesättigten Keton werden mit Hilfe des erfindungsgemäßen Verfahrens erzielt, wenn man ein Katalysatorsystem verwendet, das aus Dimethylamin und Essigsäure besteht.

Mit Vorteil verwendet man das Dimethylamin oder das Pyrrolidin in Form einer wässrigen Lösung.

Zur Herstellung des entsprechenden gesättigten Ketons, insbesondere von 2-Methyl-heptan-2-on, kann man das nach dem erfindungsgemäßen Verfahren erhaltene, das ungesättigte Keton der allgemeinen Formel I enthaltende Reaktionsgemisch nach Abtrennung der gebildeten wäßrigen Phase ohne weitere Aufarbeitung in an sich bekannter Weise zu dem entsprechenden gesättigten Keton hydrieren.

Als für das erfindungsgemäße Verfahren geeignete Ketone seien insbesondere Aceton, Methylethylketon und Cyclohexanon genannt, als geeignete Aldehyde insbesondere Propionaldehyd, Butyraldehyd, Valeraldehyd, Isovaleraldehyd, Citronellal, 3-(tert.-Butyl-phenyl)-2-methyl-propanal, 3-(Methyl-phenyl)-2-methyl-propanal und 3-(Methoxy-phenyl)-2-methyl-propanal, insbesondere Isover-aldehyd und Citronellal.

Als geeignete Amine seien insbesondere sekundären Amine wie Pyrrolidin und Dimethylamin, insbesondere das Dimethylamin, genannt.

Als Carbonsäure kommen vorzugsweise Carbonsäuren mit 2 bis 10 C-Atomen in Betracht. Hierbei ist es unerheblich, ob eine aliphatische oder eine aromatische Carbonsäure verwendet wird. Es können auch Carbonsäuren, die 2 oder mehrere Carboxylgruppen enthalten, wie Adipinsäure oder Phthalsäure verwendet werden. Mit besonderem Vorteil verwendet man Essigsäure, Phthalsäure und Adipinsäure, insbesondere Essigsäure.

Die Reaktion kann unter Atmosphärendruck oder unter höheren Drukken ausgeführt werden. Im allgemeinen reicht jedoch Atmosphärendruck aus.

Die Temperatur kann über einen weiten Bereich zwischen etwa -10 bis 200°C variiert werden. Wird beispielsweise Aceton als Keton-komponente verwendet, so wird die Reaktion bevorzugt bei der Temperatur ausgeführt, bei der das vorgelegte Aceton siedet.

Das erfindungsgemäße Verfahren kann sowohl diskontinuierlich, als auch halbkontinuierlich oder kontinuierlich durchgeführt werden.

Das neue Verfahren ist besonders geeignet zu Herstellung von 6-Methyl-heptan-2-on, wobei zunächst Aceton in Gegenwart eines Katalysators bestehend aus wässrigem Dimethylamin und Essigsäure mit Isovaleraldehyd zur Reaktion gebracht wird und das Reaktionsprodukt, das nach Abtrennung der gebildeten wäßrigen Phase im wesentlichen aus 6-Methyl-3-hepten-2-on (MHE) besteht, in an sich bekannter Weise an einem Hydrierkatalysator mit Wasserstoff zu MHA hydriert wird.

Wie das Beispiel 15 zeigt, kann der von der gebildeten wäßrigen Phase befreite Kondensationsaustrag an einem üblichen Hydrierkatalysator, wie einem Katalysator enthaltend 5-Gew.-% Pd auf Aktivkohle, bei 3 bis 20 °C und ca. 1 bar Wasserstoffdruck, also unter sehr milden Hydrierbedingungen, mit quantitativem Umsatz hydriert werden.

Als übliche Hydrierkatalysatoren sind beispielsweise Katalysatoren geeignet, die mindestens ein Element der 8. bis 10. Nebengruppe des Periodensystems enthalten, vorzugsweise Ni oder Co, oder aber ein Edelmetall, wie Pt, Pd, Rh, Ru, Ir, Au oder Ag, insbesondere Pd. Die Hydrierkatalysatoren können in metallischer oder oxidischer Form auf inerten Trägermaterialien, wie Kohle, SiO₂, TiO₂, ZrO₂ oder Al₂O₃ oder aber in Träger-loser Form, wie das beispielsweise bei Verwendung von Raney-Nickel oder Raney-Cobalt der Fall ist, eingesetzt werden.

Die Hydrierung kann unter den üblichen Bedingungen bei einem Wasserstoffüberdruck von 0,5 bis 50 bar, vorzugsweise bei 1 bis 10 bar und Temperaturen von 0 bis 100°C, vorzugsweise von 20 bis 40°C durchgeführt werden.

Die Aufarbeitung kann auf der Stufe des ungesättigten Ketons oder nach der Hydrierung mit Hilfe der üblichen Methoden, zum Beispiel durch Destillation oder Kristallisation erfolgen.

MHA ist ein wichtiges Vorprodukt zur Herstellung von fettlöslichen Vitaminen, wie Vitamin-E. In der wissenschaftlichen Literatur existiert eine Vielzahl von Synthese-methoden, die u.a. in EP 816 321 genannt werden. Als Nachteil dieser Verfahren ist oft das schlechte Verhältnis von Selektivität und Raum-Zeit-Ausbeute zu nennen.

Durch das erfindungsgemäße Verfahren kann 6-MHA mit Selektivitäten > 92 %, bezogen auf den im Unterschuß eingesetzten Aldehyd in guten Raum-Zeit-Ausbeuten hergestellt werden.

Auch das für die Herstellung von verschiedenen Wirkstoffen geeignete und daher begehrte 6,10-Dimethyl-undeca-3,9-dien-2-on sowie 6-(tert.-Butyl-phenyl)-5-methyl-hexan-2-on können mit unerwartet hohen Selektivitäten erhalten werden.

Ferner lassen sich die aus der Kondensation hevorgegangenen Ketone je nach Art des eingesetzten Katalysators und den gewählten Reaktionsbedingungen zu den entsprechenden Alkoholen umsetzen.

### Beispiele 1 bis 6 und Vergleichsbeispiele 1* bis 5*

### Allgemeine Versuchsdurchführung

Die Versuche wurden in einer 250 ml Rührapparatur mit aufgesetztem Rückflußkühler und Tropftrichter nach folgender Standardvorschrift durchgeführt:

### Allgemeine Verfahrensdurchführung:

In die Rührapparatur wurden 113 g (1,95 mol) Aceton und die in der Tabelle angegebene Menge Katalysator (angegeben in Mol-%, bezogen auf den eingesetzten Aldehyd) unter Rühren bis zum Rückfluß erhitzt. Anschließend wurde zu diesem siedenden Gemisch 0,65 mol Isovaleraldehyd (IVA) innerhalb von 5 bis 6 Stunden (h) bei Normaldruck zugetropft und das Reaktionsgemisch nach Beendigung des Zutropfens noch 1 h nachgerührt. Die Auswertung der Versuche erfolgte durch gaschromatographische Analyse (GC) auf Basis der GC-Flächenprozente (Methode: 50 m OV 1701, 25 µm, 50/10/240), wobei Aceton als Lösungsmittel bewertet wurde und nicht in die Auswertung mit einging. Für die Berechnung von Umsatz und Selektivität wurde die Summe der Wertprodukte (WP) aus den Reaktionsprodukten 6-MHE, dessen dekonjugierten Isomeren und dem β-Hydroxy-keton (6-MHOL) gebildet.

### 1) Einfluß des verwendeten Amins

Für das Katalysatorsystem wurden als Aminkomponenten Dimethylamin (DMA), Diethylamin (DEA), Pyrrolidin (Pyrr), Piperidin (Pip) und Morpholin (Morph) in Gegenwart von Essigsäure getestet. Hierzu wurden bei der Herstellung der Katalysatorsysteme alle Amine in Form einer 40 Gew.-%-igen Lösung in Wasser vorgelegt und hierzu unter Kühlung Essigsäure zugetropft. Im Vergleichsbeispiel 5* wurden Diethanolamin (DIEA) und Oxalsäure (Oxals) als Katalysator eingesetzt. Im Vergleichsbeispiel 4* wurde nur wäßrige DMA-Lösung ohne Säure eingesetzt. Die Ergebnisse der durchgeführten Versuche sind in Tabelle 1 zusammengefaßt.

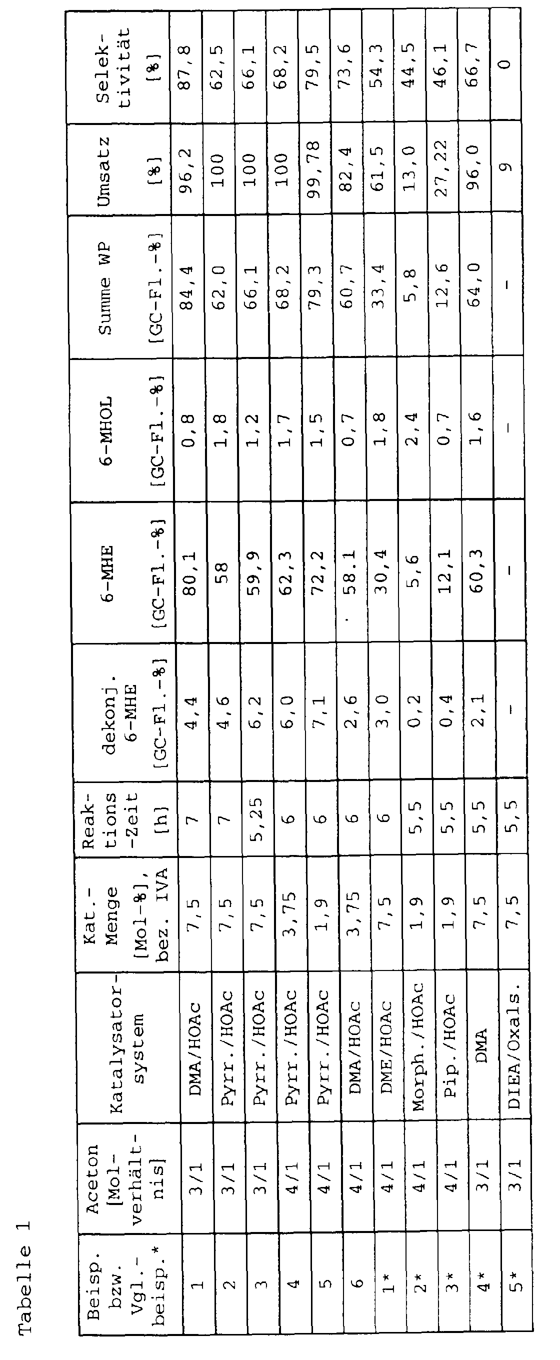

Die Ergebnisse aus Tabelle 1 zeigen, daß mit dem Katalysatorsystem Dimethylamin/Essigsäure bei nahezu vollständigem Umsatz die größten Selektivitäten erreicht werden können, wenn der Katalysator in Mengen von etwa 7,5 Mol-% zum Isovaleraldehyd IVA eingesetzt wird. Alle anderen Amin-Carbonsäure-Kombinationen liefern schlechtere Selektivitäten bzw. versagen, wie im Falle des Systems Diethanolamin/Oxalsäure ganz.

### 2) Variation der Brönstedtsäure

### Beispiele 7 bis 9 und Vergleichsbeispiele 6* bis 19*

Für diese Versuche wurde Dimethylamin als Base eingesetzt. Als Brönstedtsäuren wurden als organische Säuren Essigsäure, Adipinsäure, Phthalsäure und Oxalsäure eingesetzt, außerdem die sauren Ionenaustauscher Dowex 50 WX8, Lewatit SPC 112/H, und als anorganische Säuren Amberlist 15, Amberlit IR, Deloxan, β-Zeolith GE 1494, Montmorillonit KSF, Al₂O₃ und Bentonit K-10 verwendet. Die Menge der heterogenen Säuren wurde auf 0,5 g festgelegt und in Vorversuchen die zur Neutralisation erforderliche Menge an 40 %-igem wässrigen DMA bestimmt.

Die löslichen Katalysatorssyteme (DMA/Adipinsäure, DMA/Phthalsäure und DMA/Oxalsäure) wurden, wie oben für das Amin angegeben, im Verhältnis von 7,5 Mol-%, bezogen auf den eingesetzten Aldehyd IVA verwendet. Das Verhältnis von Aceton zu IVA wurde auf 3:1 eingestellt.

Die erzielten Ergebnisse sind in Tabelle 2 dargestellt.

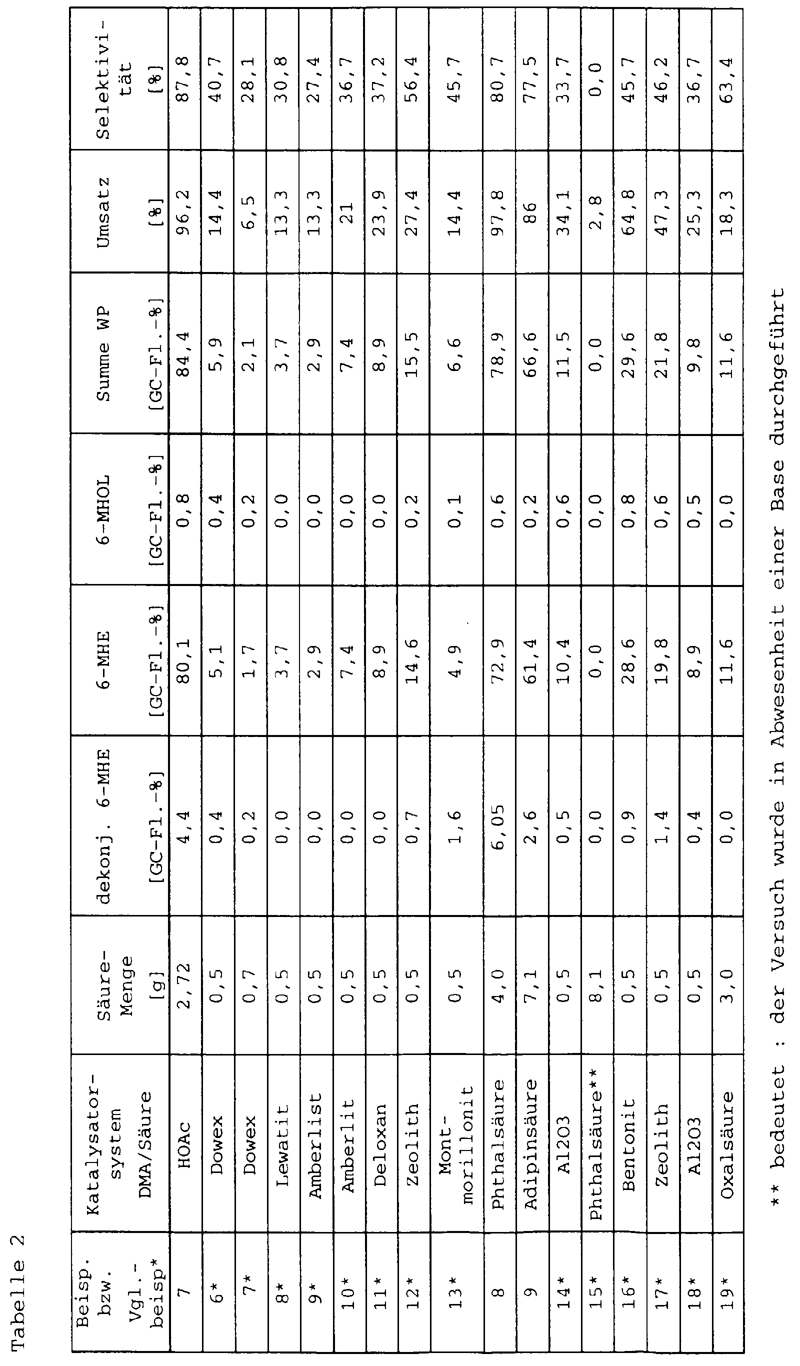

Aus den in Tabelle 2 dargestellten Ergebnissen wird die überraschende deutliche Überlegenheit der in Wasser löslichen Katalysatorsysteme gegenüber den partiell heterogenen Katalysatorsystemen ersichtlich, wobei sich Essigsäure als beste Säurekomponente erwies. Essigsäure wurde daher auch für die folgenden Versuche eingesetzt. Alternativ sind aber auch Phthalsäure und Adipinsäure für den Kondensationsschritt gut geeignet.

In Vergleichsversuch 15* wurde vergeblich versucht, die Kondensation in Abwesenheit eines Amins, also nur in Gegenwart von Phthalsäure durchzuführen.

### Beispiel 10 und Vergleichsbeispiele 20* bis 23*

Jeweils 70 g Aceton wurden bei Raumtemperatur (Rt) zusammen mit der aus der folgenden Tabelle 3 ersichtlichen Menge an DMA und der in der Tabelle 3 angegebenen Säure in der dort angegebenen Menge (entsprechend 7,5 Mol-% Katalysatorsystem) in einer Rührapparatur vorgelegt und unter Rühren erhitzt. Nach Erreichen der Rückflußtemperatur wurden innerhalb von 5 h 34,4 g IVA, gelöst in 46,5 g Aceton, zugetropft. Die Reaktionsausträge wurden mittels GC analysiert.

**Tabelle 3**

| Beispiel bzw. Vergleichsbeispiel* | Menge Dimethylamin | Menge Säure | Umsatz an IVA | Ausbeute an 6-MHE |
|---|---|---|---|---|
| 10 | 1,32 g | 1,8 g Essigsäure | > 99 % | 84,2 % |
| 20* | 1,32 g | 1,38 g Ameisensäure | 21 % | 13,0 % |
| 21* | 1,32 g | 3,0 g Phosphorsäure | 0 % | 0 % |
| 22* | 2,64 g | 3,0 g Phosphorsäure | 35 % | 15,6 % |
| 23* | 3,96 g | 3,0 g Phosphorsäure | 70 % | 35,4 % |

Aus den Vergleichsversuchen 20* bis 23* geht hervor, daß die Selektivitäten an 6-MHE mit den Säuren Ameisensäure oder Phosphorsäure im Katalysatorsystem wesentlich schlechter sind, als mit Essigsäure.

### 3) Bestimmung des Einflusses von Zeit, Wassergehalt im Katalysatorsystem und dem Aceton/IVA-Verhältnis auf die Selektivität

### Beispiele 11 bis 14

Als Katalysatorsystem wurde DMA/Essigsäure nach der bereits oben beschriebenen Standardvorschrift (je 7,5 Mol-%), jedoch bei einem Aceton/IVA-Verhältnis von 5 : 1 eingesetzt, wobei 40 % des Acetons zum Vorverdünnen des IVA verwendet wurden. Die Ergebnisse wurden in Tabelle 4 dargestellt.

Die in Tabelle 4 dargestellten Ergebnisse zeigen, daß sich durch die Erhöhung des Aceton/IVA-Verhältnisses auf 5/1 die Selektivität der Aldolkondensation auf über 92 % steigern läßt.

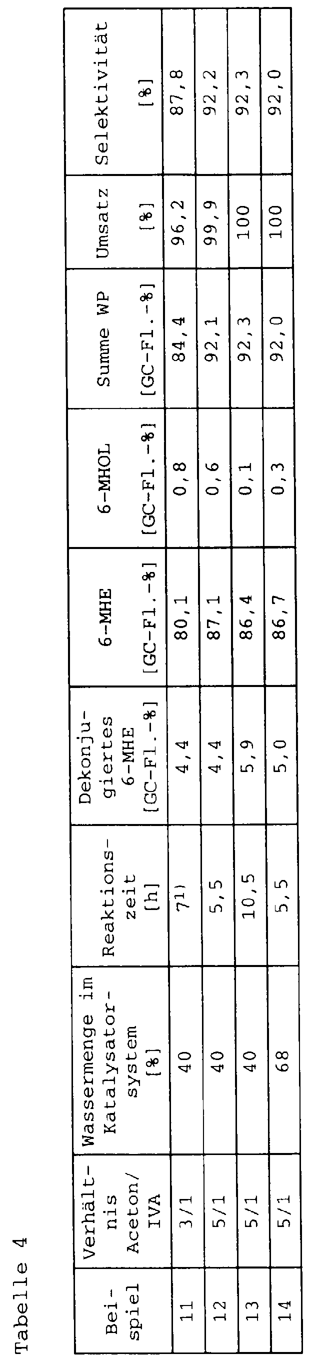

### 4) Hydrierung des Rohaustrages

### Beispiel 15

100 g des Reaktionsaustrags aus Beispiel 12 (s. Tabelle 4) wurde nach Abtrennung der in der Kondensationsreaktion anfallenden wäßrigen Phase ohne weitere Aufarbeitung in Gegenwart von 2 g eines Hydrierkatalysators enthaltend 5 Gew.-% Pd auf Kohle bei 20°C und ca. 1 bar Wasserstoffdruck hydriert. Nach 2,5 h kam die Wasserstoffaufnahme zum Erliegen. Die GC-Analyse des Hydrieraustrages bestätigte den quantitativen Umsatz von 6-MHE in das gewünschte 6-Methyl-heptanon. Dieser Versuch dokumentiert, daß es prinzipiell möglich ist, 6-MHE unter sehr milden Bedingungen mit unerwartet hohen Selektivitäten zum gesättigten Keton zu hydrieren.

### Beispiel 16

150 g Aceton wurden bei Rt zusammen mit 1,32 g Dimethylamin und 1,8 g Essigsäure (entsprechend 7,5 Mol-%, bezogen auf das Katalysatorsystem) in einer Rührapparatur vorgelegt und unter Rühren erhitzt. Nach Erreichen der Rückflußtemperatur wurden innerhalb von 5 h 100 g Citronellal zu dem siedenden Reaktionsgemisch zugetropft. Die Reaktionsausträge wurden mittels GC analysiert. Der Umsatz an Citronellal betrug 84 %, die Selektivität an 6,10-Dimethyl-undeca-3,9-dien- 2-on betrug 95 % der Theorie.

## Patentansprüche

1. Verfahren zur Herstellung von α,β-ungesättigten Ketonen der allgemeinen Formel I 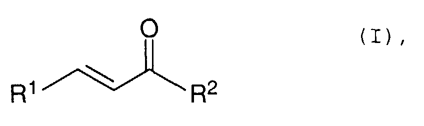 in der R¹ für einen verzweigten oder unverzweigten cyclischen oder acyclischen gesättigten oder ungesättigten aliphatischen oder araliphatischen Rest mit 4 bis 20 C-Atomen steht, und R² für einen verzweigten oder unverzweigten cyclischen oder acyclischen gesättigten oder ungesättigten aliphatischen oder araliphatischen Rest mit 1 bis 10 C-Atomen steht, durch Umsetzen von einem Keton der allgemeinen Formel II 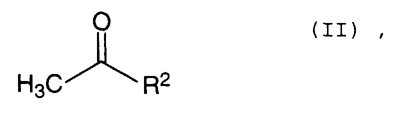 in der R² die oben angegebene Bedeutung hat, mit einem Aldehyd der allgemeinen Formel III 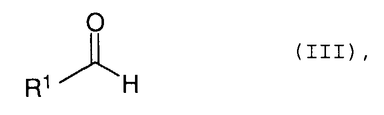 in der R¹ die oben angegebene Bedeutung hat, dadurch gekennzeichnet, daß man das Keton in molarem Überschuß in Gegenwart von einem Katalysatorsystem bestehend aus Dimethylamin oder Pyrrolidin und einer Carbonsäure enthaltend mindestens 2 C-Atome unter Rückfluß zum Sieden erhitzt und zu dieser Mischung langsam den Aldehyd der allgemeinen Formel III zufügt.

2. Verfahren gemäß Anspruch 1, dadurch gekennzeichnet, daß man in dem Katalysatorsystem das Dimethylamin oder das Pyrrolidin und die Carbonsäure in etwa äquimolaren Mengen einsetzt.

3. Verfahren gemäß Anspruch 1, dadurch gekennzeichnet, daß man das Katalysatorsystem in Mengen von etwa 5 bis 10 Mol-%, bezogen auf den eingesetzten Aldehyd der allgemeinen Formel III verwendet.

4. Verfahren gemäß Anspruch 1, dadurch gekennzeichnet, daß man das Katalysatorsystem in Mengen von etwa 7 bis 8 Mol-%, bezogen auf den eingesetzten Aldehyd der allgemeinen Formel III verwendet.

5. Verfahren gemäß Anspruch 1, dadurch gekennzeichnet, daß das Katalysatorsystem aus Dimethylamin oder Pyrrolidin und einer Carbonsäure enthaltend 2 bis 10 C-Atome besteht.

6. Verfahren gemäß Anspruch 1, dadurch gekennzeichnet, daß das Katalysatorsystem aus Dimethylamin und Essigsäure besteht.

7. Verfahren gemäß Anspruch 1, dadurch gekennzeichnet, daß man das Dimethylamin oder das Pyrrolidin in Form einer wäßrigen Lösung verwendet.

8. Verfahren gemäß Anspruch 1, dadurch gekennzeichnet, daß man das erhaltene ungesättigte Keton der allgemeinen Formel I nach Abtrennen der gebildeten wäßrigen Phase ohne weitere Aufarbeitung in an sich bekannter Weise in das entsprechende gesättigte Keton hydriert.

9. Verfahren gemäß Anspruch 1, dadurch gekennzeichnet, daß man das Keton in einem mindestens 2-molaren Überschuß gegenüber dem Aldehyd der Formel III verwendet.

10. Verfahren gemäß Anspruch 1, dadurch gekennzeichnet, daß man zur Herstellung von 6-Methyl-heptan-2-on Isovaleraldehyd mit Aceton in einem 4,5- bis 5,5-fach molarem Überschuß in Gegenwart von einem Katalysatorsystem bestehend aus etwa äquimolaren Mengen an Dimethylamin und Essigsäure in Mengen von etwa 6 bis 8 Mol-%, bezogen auf den Isovaleraldehyd, umsetzt und das erhaltene 6-Methyl-3-hepten-2-on in an sich bekannter Weise katalytisch hydriert.
